# EUROPEAN PATENT APPLICATION

(11) **EP 4 609 728 A1**
(43) Date of publication of application: **03.09.2025**
(21) Application number: 25161416.0
(22) Date of filing: 03.03.2025
(51) Int. Cl.: A23L 33/105, A23L 33/16, A61K 36/185, A61K 36/49, A61K 36/736, A61K 33/00, A61K 41/00

(54) **METHOD FOR OBTAINING A NUTRACEUTICAL COMPOSITION AND NUTRACEUTICAL COMPOSITION OBTAINED THEREBY**

(30) Priority: 01.03.2024 IT 202400004666
(71) Applicant: Sitar Italia S.r.l., 35135 Padova (PD) (IT)
(72) Inventor: GIARRIZZO, David, 35135 PADOVA (PD) (IT); MAIOLO, Luigi, 12040 BALDISSERO D'ALBA (CN) (IT); STRADA, Mario Napoleone, 21047 SARONNO (VA) (IT)
(74) Representative: Morabito, Sara

(57) **Abstract**

A method for obtaining a nutraceutic composition comprises the step of providing an extraction solution containing approximately from 29.9 to 69.9% by weight water and approximately from 29.9 to 69.9% by weight of at least one alcohol which is selected from a group comprising ethyl alcohol, propyl alcohol, glycerine or the combinations thereof and approximately from 0.001% to 0.1% by weight of at least one biochemical salt, said biochemical salt being selected from a group comprising:
*Salt no. 1 Calcium fluoratum -* calcium fluoride, CaF2;
Salt no. 2 *Calcium phosphoricum -* calcium acid phosphate CaHPO4;
Salt no. 3 *Calcium sulfuricum -* Calcium sulphate CaSO4
Salt no. 4 *Ferrum phosphoricum -* iron pyrophosphate Fe₄(P₂O₇)₃;
Salt no. 5 *Kalium chloratum -* potassium chloride KCl;
Salt no. 6 *Kalium phosphoricum -* potassium phosphate K3PO4;
Salt no. 7 *Kalium sulfuricum -* potassium sulphate K2SO4;
Salt no. 8 *Magnesium phosphoricum* - magnesium phosphate MgHPO4;
Salt no. 9 *Natrium chloratum* - sodium chloride NaCl;
Salt no. 10 *Natrium phosphoricum -* sodium phosphate Na3PO4;
Salt no. 11 *Natrium sulfuricum -* sodium sulphate Na2SO4;
Salt no. 12 Silicea - silicon dioxide SiO2;
or the combinations thereof;
- immersing in said extraction solution a quantity of at least one plant meristem so as to obtain an admixture having a weight ratio of meristem:extraction solution between 1:3 and 1:20;
- leaving said plant meristems to macerate in said extraction solution for a maceration period between 20 hours and 3 weeks so as to extract one or more bioactive compounds from said at least one plant meristem,
- filtering said admixture so as to obtain a nutraceutic composition comprising between approximately from 30 to 69.6% by weight of water, approximately from 30 to 69.6% by weight of said at least one alcohol, approximately from 0.001 to 0.1% by weight of said salt and approximately from 0.3 to 5% by weight of said one or more bioactive compounds.

## Description

The invention concerns a method for obtaining a nutraceutic composition and the relative nutraceutic composition. The invention further concerns a food supplement based on such a nutraceutic composition.

The food supplement can be in the form of a liquid preparation to be administered in drops, drink, syrup, tablets. The invention further concerns a beverage or syrup containing such a nutraceutic composition. The invention further relates to a tablet or capsule obtained from said nutraceutic composition.

The invention further concerns a cosmetic product in the form of a cream or gel obtained from said nutraceutic composition.

The invention further concerns a product obtained from said nutraceutic composition, said product being selected from a group comprising a cream, tablet, capsule, patch, beverage.

The nutraceutic composition contains derivatives of plant origin. Derivatives of plant origin are derived in particular from meristems or embryonic parts of plants.

Compounds derived from plant sources, also known as phytotherapeutic products, are widespread and can be used as food supplements or even as nutraceutical formulations that can be used in cosmetic formulations or for cosmetic purposes.

In recent decades, food supplements have had a major impact on consumers who are showing an increasing interest in preserving their health and their well-being.

In this panorama, particular attention has been paid to bud therapy, which is based on the use of particular parts of plants, referred to as embryos or meristems, such as, for example, buds, roots, catkins, young buds, young branches and seeds. Bud-derivatives (BD) are concentrated sources of nutrients or bioactive compounds with nutritional or physiological effects and, due to their purported health benefits, can be used as food supplements. Bud-derivatives contain embryonic tissue of the plants, which in turn contain particular totipotent cells, called meristems. These cells, in fact, have the ability to operate mitosis continuously, even to modulate cell regeneration and response in situations of damage and, therefore, have a great resistance to apoptosis.

In order to support the metabolic activity of the meristems, the embryonic tissues of the plants are endowed with various complexes of "active" substances (or even "active" principles) that are necessary for the development of the adult plant in its various parts. Such active substances comprise minerals in ionic form, vitamins, amino acids, phytohormones, phenolic acids, nucleic acids, enzymes, trace elements, flavonoids, sesquiterpenes and tannins, as well as carbohydrates, lipids and proteins. These compounds, in addition to promoting the development of the plants, perform an immune defence-supporting function, that is, they protect it from external agents, and an antioxidant action for the plant.

For this reason, several methods have been tested to "capture" the vitality of the meristem tissue and transfer it into a technological product for improving health and supporting human and veterinary drug treatments.

"Bud-derivatives" are a relatively new category of herbal food supplements that are conventionally produced, according to the European Pharmacopoeia 8th edition, by cold maceration of plant meristems, of fresh meristematic tissues coming from trees and/or herbaceous plants, buds, young shoots, lymphs, catkins.

It is usually known to use admixtures of water, ethanol and glycerol as extraction solvents.

Although the use of bud-derivatives has been known for a very long time as food supplements, however, the European Food Safety Authority (EFSA) has not yet approved any specific indication regarding the therapeutic effects or in general the effects on human health for the bud-derivatives other than limited to some of said bud-derivatives.

On the other hand, the composition of such bud-derivative products can be largely influenced not only by the type of plant used but also by the specific part of the plant that is used or also by the cultivation and harvesting area or even by the process used to obtain the bud-derivative. All these factors influence the final composition of the bud-derivative in qualitative and quantitative terms.

On the one hand, it is necessary to comply with certain protocols for the production of the bud-derivative in order to ensure the presence of desired substances within it. On the other hand, however, it is necessary to ensure that the desired substances are present at desired concentration levels or concentration ranges for the desired effects to be obtained.

For example, in recent years, many benefits for human health have been described from the use of dietary supplements containing polyphenols, for example to combat ageing, the onset of cardiovascular diseases, to prevent obesity and diabetes, to modulate the human gut microbiota and to improve the cognitive abilities of the brain. However, the polyphenol content in a bud-derivative is strongly influenced by the production methods of the bud-derivatives, e.g. solvent ratios in the extraction mixtures, raw material/extraction admixture ratios, extraction time as well as the botanical source used.

Similar considerations can also be extended to other compounds found in the bud-derivatives.

There remains therefore a need to provide a bud-derivative having a predictable composition.

Bud-derivatives are currently obtained through two main technologies: cold maceration and pulsed ultrasound-assisted extraction, following two different experimental production protocols.

In cold maceration, it is provided for the buds to macerate for 21 days in glycerol/ethanol 96% (1/1 w/w) with a desired buds/solvent ratio, according to the official method for glyceric macerates reported in the European Pharmacopoeia 8th edition.

Pulsed ultrasound-assisted extraction allows bud-derivatives to be obtained faster than maceration. Furthermore, the ultrasound-assisted extraction allows to obtain a reduction in the extraction times, decrease in the solvent used and in the energy used, however, it does not allow to obtain a bud-derivative having a desired composition. Sometimes this extraction system damages the quality of the buds used.

At the end of the extraction procedure an admixture is obtained which is filtered to remove the solid part and a liquid composition is obtained comprising a solvent and compounds of plant origin dispersed in the solvent.

Another problem deriving from the known bud-derivatives is that the substances present in the bud-derivatives are currently not always directly usable by the body in an effective manner. With the known bud-derivatives it is necessary for the user to take high quantities of such products to obtain an effective effect, especially in the short term.

There remains therefore the need to provide a bud-derivative in which the substances are highly bioavailable and can be easily absorbed by the body.

It is necessary to provide an effective bud-derivative that allows it to exert its properties even if taken at limited concentrations and that enables its beneficial effects to be exerted in the short term. There still remains a need to provide a bud-derivative that allows it to show its beneficial effects after a reduced period of time from its intake by the user.

The use of Schüssler salts, i.e. the 12 inorganic salts identified by the German physician Wilhelm Heinrich Schüssler that are present in the human organism and that play an important role in cell physiology, is also well known in the field of natural medicine. The human body is composed of a group of essential salts (also known as biochemical salts) whose imbalance in the human tissues can compromise the general state of health. Therefore, restoring balanced levels of these salts in the body leads to an improvement in the general state of health. These salts help to allow the proper functioning of the cells, regulate and improve the bioavailability of the endogenous salts that are essential for cellular biochemical metabolic processes and help to restore the relative deficiencies of the biochemical salts, a potential cause of the onset of malaises.

However, in order to be properly and effectively assimilated, these biochemical salts must be provided in a suitable formulation that makes them bioavailable and easily assimilable by the body.

There remains therefore the need to provide food supplements containing one or more of the Schüssler Salts that are highly assimilable by the body.

The Applicant has understood the need to limit the number of supplements to be taken daily by the users.

The Applicant has ascertained the need on the part of the consumers to have a food supplement and in particular a compound based on plant products and in particular on bud-derivatives that is effective even if taken in limited doses and that allows its beneficial effects to be exerted in the short term.

The Applicant speculated that the simultaneous intake of bud-derivatives and of biochemical salts could influence the absorption of the bud-derivatives and of the salts themselves.

The Applicant therefore considered adding one or more Schüssler salts into a bud-derivative among those commercially available.

However, the Applicant verified that this expedient did not allow to improve the characteristics of the bud-derivative and that this expedient did not allow to appreciably improve the absorption by the body or the bioavailability of the phytotherapeutic compounds.

Furthermore, the Applicant verified that this expedient did not allow to completely overcome the limits of the known bud-derivatives.

The Applicant therefore verified that the addition of one or more Schüssler salts during the process of extraction of the bioactive compounds from the meristem products allowed to improve the extraction of the bioactive compounds contained in the plant meristems from the meristem products themselves, allowing to obtain a composition having a higher concentration of plant bioactive compounds under the same conditions. In particular, the Applicant verified that adding one or more Schüssler salts during the maceration step of the plant meristems allowed to improve the extraction of the plant bioactive compounds contained in the meristemic products themselves, actually obtaining a more concentrated composition in bioactive compounds coming from the plant meristemic products.

The Applicant also verified that the addition of a specific Schüssler salt during the process of extraction of the bioactive compounds from the meristemic products selected according to the particular meristemic material allowed to improve the extraction of the bioactive compounds contained in the plant meristems from the meristemic products themselves, allowing to obtain a composition having a higher concentration of plant bioactive compounds under the same conditions. In other words, the Applicant verified that the various Schüssler Salts were particularly effective at extracting particular meristemic materials, enhancing the extraction of the actives contained therein. The Applicant also verified that this effect could be obtained without damaging the meristemic material during the maceration step.

The Applicant verified that by adding a Schüssler salt during the maceration step of the plant meristems it was possible to increase the extraction from the plant meristems of amino acids, polyphenols and antioxidants contained in the plant meristems.

Within the scope of the present description some definitions assume the meaning indicated below.

By alcohol is meant an organic compound that contains at least one hydroxyl functional group (-OH) bonded to a saturated carbon atom. Alcohols can be monofunctional, i.e. have a single hydroxyl group or polyfunctional, also called polyols or polyalcohols, when they are provided with two or more alcohol groups.

By the term biochemical salt is meant one of the Schüssler Salts, i.e. a salt selected from calcium fluoride, CaF₂; calcium acid phosphate, CaHPO₄; Calcium sulphate, CaSO₄; iron pyrophosphate, Fe₄(P₂O₇)₃; potassium chloride, KCl; potassium phosphate, K₃PO₄; potassium sulphate, K₂SO₄; magnesium phosphate, MgHPO₄; sodium chloride, NaCl; sodium phosphate, Na₃PO₄; sodium sulphate, Na₂SO₄; silicon dioxide, SiO₂.

By plant meristems are meant the plant derivatives from buds, young buds, lymphs, catkins and other. In particular, plant meristems refer to fresh plant tissues or juvenile parts of the plants, such as buds, shoots, young roots, twigs and their bark, as well as any other tissue that somehow contains meristems or developing cells, such as plant embryos, i.e. seeds. The term plant meristems includes herein also the lymph which, although it is not really a shoot or a bud, contains a large quantity of active substances that can be used in bud therapy. The meristematic tissues contain a population of small totipotent cells, characterized by a dense cytoplasm and by a large nucleus. These cells are mitotically active, that is, they have the ability to divide by mitosis to give rise to two cells. Of the latter, one remains meristematic, while the other undergoes differentiation based on its role within the plant body. Therefore, from the germinating seed to the mature stage, the development of the plant body depends on the activity of the meristems.

Bud-derivative means a composition derived from the processing of buds or in general of plant meristems. The bud-derivatives are obtained from young and developing plant tissues: buds, shoots, rootlets.

Phytotherapeutic means a compound obtained starting from the adult parts of the plant (leaves, roots, flowers, etc.).

The term bud therapy refers to a branch of phytotherapy that uses tissues and substances present in the young parts of the plants.

The term active substances or active ingredients or bioactive composites is intended to indicate nutraceutic substances that can promote the proper functioning of an organism, participating in cellular metabolic and enzymatic processes. The active substances may comprise mineral salts and organic substances processed by the plants themselves including simpler compounds such as sugars, amino acids and lipids and more complex compounds such as terpenes, flavonoids and alkaloids.

The term bud-derivative refers to the extract of plant meristems by an extraction solution.

A nutraceutic composition is intended as a food composition suitable for ingestion and intake by human or animal organisms capable of positively acting on the physiological functions of the organism and of favouring the well-being thereof by counteracting degenerative processes.

According to a first aspect of the invention, a method is provided for obtaining a nutraceutic composition comprising the step of providing an extraction solution containing approximately from 29.9 to 69.9% by weight of water and approximately from 29.9 to 69.9% by weight of at least one alcohol which is selected from a group comprising ethyl alcohol, propyl alcohol, glycerine or the combinations thereof and approximately from 0.001% to 0.1% by weight of at least one biochemical salt, said biochemical salt being selected from a group comprising:
*Salt no. 1 Calcium fluoratum -* calcium fluoride, CaF₂;
Salt no. 2 *Calcium phosphoricum -* calcium acid phosphate CaHPO₄;
Salt no. 3 *Calcium sulfuricum -* Calcium sulphate CaSO₄;
Salt no. 4 *Ferrum phosphoricum -* iron pyrophosphate Fe₄(P₂O₇)₃;
Salt no. 5 *Kalium chloratum -* potassium chloride KCl;
Salt no. 6 *Kalium phosphoricum -* potassium phosphate K₃PO₄;
Salt no. 7 *Kalium sulfuricum -* potassium sulphate K₂SO₄;
Salt no. 8 *Magnesium phosphoricum* - magnesium phosphate MgHPO₄;
Salt no. 9 *Natrium chloratum* - sodium chloride NaCl;
Salt no. 10 *Natrium phosphoricum -* sodium phosphate Na₃PO₄;
Salt no. 11 *Natrium sulfuricum -* sodium sulphate Na₂SO₄;
Salt no. 12 Silicea - silicon dioxide SiO₂;
or any combinations thereof.

In a preferred version said extraction solution comprises a single biochemical salt selected from Schüssler salts.

In a preferred version, said method comprises preparing an extraction solvent comprising from 30 to 70% by weight water and approximately from 30 to 70% by weight of one at least one alcohol selected from a group comprising ethyl alcohol, propyl alcohol, glycerine and dissolving in said solvent approximately from 0.001% to 0.1% by weight of one biochemical salt in order to obtain said extraction solution.

Preferably said method comprises the step of dissolving in said solvent approximately from 0.001% to 0.1% by weight of a biochemical salt selected from Schussler salts.

The extraction solution preferably consists of water, a monofunctional alcohol, such as for example ethyl alcohol or propyl alcohol, glycerine and a biochemical salt. Preferably the extraction solution consists of approximately from 29.9 to 69.9% by weight water, approximately from 29.9 to 69.9% by weight of an alcohol selected from ethyl alcohol or propyl alcohol, approximately from 29.9 to 69.9% by weight glycerine and approximately from 0.001% to 0.1% by weight of a biochemical salt.

Preferably, the biochemical salt is selected as a function of the plant meristem to be immersed in the extraction solution.

Preferably said method comprises the step of immersing in said extraction solution a desired quantity of at least one plant meristem so as to obtain an admixture having a weight ratio meristem: extraction solution between approximately 1:3 and approximately 1:20, preferably between approximately 1:10 and approximately 1:20, preferably between approximately 1:15 and 1:20.

The weight ratio between meristem: extraction solution is calculated considering the dry content of the plant meristems used. In this way, dilution of the extraction solution is avoided and an extraction solution having a more precise composition and not dependent on the characteristics of the individual meristem crop is obtained.

Preferably said method comprises a maceration step in which it is provided for leaving said at least one plant meristem macerate in said extraction solution for a maceration period between 20 hours and approximately 3 weeks so as to extract one or more bioactive compounds from said at least one plant meristem.

Preferably said method comprises a maceration step in which it is provided for leaving said at least one plant meristem macerate in said extraction solution for a maceration period from 2 weeks to 4 weeks, preferably approximately 3 weeks, so as to extract one or more bioactive compounds from said at least one plant meristem.

In one version said method comprises a maceration step in which it is provided for leaving said at least one plant meristem macerate in said extraction solution for a maceration period of at least 3 weeks, so as to extract one or more bioactive compounds from said at least one plant meristem.

Preferably, said method comprises the step of filtering said admixture so as to obtain a nutraceutic composition comprising between approximately from 30 to 69.6% by weight of water, approximately from 30 to 69.6% by weight of said at least one alcohol, approximately from 0.001 to 0.1% by weight of said salt and approximately from 0.3 to 5% by weight of said one or more bioactive compounds.

In a second aspect of the invention there is provided a nutraceutic composition comprising between approximately from 29.9 to 69.6% by weight of water, approximately from 29.9 to 69.6% by weight of at least one alcohol, approximately from 0.001 to 0.1% by weight of a biochemical salt and approximately from 0.3 to 5% by weight of one or more bioactive compounds, said nutraceutic composition being obtained by means of a method comprising the step of providing an extraction solution comprising approximately from 29.9 to 69.9% by weight water and approximately from 29.9 to 69.9% by weight of at least one alcohol selected from a group comprising ethyl alcohol, propyl alcohol, glycerine, or the combinations thereof and approximately from 0.001% to 0.1% by weight of at least one biochemical salt, said biochemical salt being selected from a group comprising:
*Salt no. 1 Calcium fluoratum -* calcium fluoride, CaF₂;
Salt no. 2 *Calcium phosphoricum -* calcium acid phosphate CaHPO₄;
Salt no. 3 *Calcium sulfuricum -* Calcium sulphate CaSO₄;
Salt no. 4*Ferrum phosphoricum -* iron pyrophosphate Fe₄(P₂O₇)₃;
Salt no. 5 *Kalium chloratum -* potassium chloride KCl;
Salt no. 6 *Kalium phosphoricum -* potassium phosphate K₃PO₄;
Salt no. 7 *Kalium sulfuricum -* potassium sulphate K₂SO₄;
Salt no. 8 *Magnesium phosphoricum* - magnesium phosphate MgHPO₄;
Salt no. 9 *Natrium chloratum* - sodium chloride NaCl;
Salt no. 10 *Natrium phosphoricum -* sodium phosphate Na₃PO₄;
Salt no. 11 *Natrium sulfuricum -* sodium sulphate Na₂SO₄;
Salt no. 12 Silicea - silicon dioxide SiO₂;
or any combinations thereof;

In a preferred version said extraction solution comprises a single biochemical salt selected from Schüssler salts.

In a preferred version, said method comprises preparing an extraction solvent comprising from 30 to 70% by weight water and approximately from 30 to 70% by weight of one at least one alcohol selected from a group comprising ethyl alcohol, propyl alcohol, glycerine and dissolving in said solvent approximately from 0.001% to 0.1% by weight of one biochemical salt in order to obtain said extraction solution.

Preferably said method comprises the step of dissolving in said solvent approximately from 0.001% to 0.1% by weight of said biochemical salt selected from Schussler salts.

. Preferably the extraction solution consists of approximately from 29.9 to 69.9% by weight water, approximately from 29.9 to 69.9% by weight of an alcohol selected from ethyl alcohol or propyl alcohol, approximately from 29.9 to 69.9% by weight glycerine and approximately from 0.001% to 0.1% by weight of a biochemical salt.

Preferably, the biochemical salt is selected as a function of the plant meristem to be immersed in the extraction solution.

Preferably said method comprises the step of immersing in said extraction solution a desired quantity of at least one plant meristem so as to obtain an admixture having a weight ratio meristem: extraction solution between 1:3 and 1:20, preferably between approximately 1:10 and approximately 1:20, preferably between approximately 1:15 and 1:20.

Preferably said method comprises a maceration step in which it is provided for leaving said at least one plant meristem macerate in said extraction solution for a maceration period between approximately 20 hours and approximately 4 weeks, preferably between approximately 2 and approximately 3 weeks so as to extract one or more bioactive compounds from said at least one plant meristem.

In a preferred version said method comprises a maceration step in which it is provided for leaving said at least one plant meristem macerate in said extraction solution for a maceration period of at least 3 weeks.

Preferably said method comprises a maceration step in which it is provided for leaving said at least one plant meristem macerate in said extraction solution for a maceration period from 2 weeks to 4 weeks so as to extract one or more bioactive compounds from said at least one plant meristem.

Preferably said method comprises the step of filtering said admixture so as to obtain said nutraceutic composition.

Thanks to the method of the invention it is possible to obtain a nutraceutic composition having improved properties compared to the known nutraceutic compositions.

By adding a biochemical salt in the solvent it is possible to obtain an extraction solution capable of improving the extraction of the bioactive compounds from the plant meristem. Further, by adding the salt to the extraction solvent, it is possible to reduce the variability of the composition of the obtained nutraceutic composition. In other words, the presence of the biochemical salt in the extraction solvent allows to control the starting composition of the plant meristem so as to reduce the variability of the nutraceutic composition obtained. The bioactive compounds are extracted from the plant meristem and are passed into the extraction solution. An admixture containing the extraction solution and the bioactive compounds extracted from the plant meristem is thus formed.

The addition of the biochemical salt to the solvent allows to increase the extraction of the protein and amino acid compounds from the plant meristem, actually favouring a greater supply of amino acids in the admixture. The extraction of phenolic or antioxidant compounds present in the plant meristem is also improved.

A nutraceutic composition having, other conditions being equal, a higher concentration of bioactive compounds, in particular of amino acid compounds is thus obtained.

A nutraceutic composition having a higher concentration of phenolic acids under other conditions is thus obtained.

A nutraceutic composition having a higher efficacy is obtained.

Moreover, any operations of concentration of the nutraceutic composition that, in addition to being expensive, could damage the bioactive composites, denaturing for example the nature of the same, are avoided.

Furthermore, the concentration range of the biochemical salt according to the invention allows to enhance the extraction of the bioactive compounds from the plant meristem without damaging the properties of the plant meristem itself.

The nutraceutic compositions of the invention are suitable for use as food adjuvants or supplements as support products for physical, mental and behavioural disorders of humans and animals.

The nutraceutic compositions of the invention can be used to support human and animal state of health in an effective manner. The nutraceutic compositions of the invention can be effectively used to intervene on metabolic processes.

The nutraceutic compositions of the invention allow to modulate and potentiate the non-specific functional reserve of the human and animal organism.

The nutraceutic compositions of the invention allow to prevent the onset of human and veterinary organ/apparatus imbalances.

The nutraceutic compositions of the invention allow to potentiate human and veterinary organ functions.

The nutraceutic compositions of the invention allow symptoms from emotional and behavioural causes to be soothed.

The nutraceutic compositions of the invention allow to cover all the structural/metabolic needs of all the symptoms.

The present invention, in at least one of the aforementioned aspects, may have at least one of the further preferred features indicated below.

The bioactive compounds contained in the nutraceutic composition of the invention are selected from a group comprising
- phenolic acids such as for example chlorogenic acid, caffeic acid, p-coumaric acid, ferulic acid, hydroxybenzoic acid;
- essential amino acids such as for example histidine, threonine, valine, methionine, phenylalanine, isoleucine, leucine, lysine;
- non-essential amino acids such as for example: proline, aspartic acid, glutamic acid, serine, glycine, arginine, alanine, tyrosine, cystine;
- Salts such as Phosphate, sulphate, Potassium, Magnesium, Calcium salts,
- Fat-soluble vitamins such as for example Vitamin K, Vitamin E;
- Water-soluble vitamins such as for example Vitamin C (Ascorbic acid),
- B vitamins such as for example Vitamin B1 (Thiamine), Vitamin B2 (Riboflavin), Vitamin B3 (Niacin), Vitamin B5 (Pantothenic Acid), Vitamin B6 (Pyridoxine), Vitamin B7 (Biotin), Vitamin B9 (Folic Acid or Folate),
- Hydrolysable tannins, Condensed tannins (or proanthocyanidins),
- Flavonoids, Flavonols, flavones,
- Terpenoids, Sesquiterpenes (e.g. β-caryophyllene) Tetraterpenes (carotenoids) such as for example β-carotene, Lutein, Zeaxanthin, Astaxanthin, Lycopene, α-carotene.

The bioactive compounds present in the nutraceutic composition will depend on the type of plant or on the types of plants used and the biochemical salt used.

In particular, the nutraceutic composition of the invention contains one or more amino acids selected from histidine, threonine, valine, methionine, phenylalanine, isoleucine, leucine, lysine, proline, aspartic acid, glutamic acid, serine, glycine, arginine, alanine, tyrosine, cystine;

Preferably said extraction solution consists of approximately from 29.9 to 69.9% by weight water and approximately from 29.9 to 69.9% by weight of at least one alcohol selected from a group comprising ethyl alcohol, propyl alcohol, glycerine, or the combinations thereof and approximately from 0.001% to 0.1% by weight of said biochemical salt.

Preferably said at least one alcohol comprises at least one polyfunctional alcohol, preferably a trifunctional alcohol, in a greatly preferred manner said at least one alcohol comprises glycerine.

Preferably said at least one alcohol comprises at least one monofunctional alcohol and at least one polyfunctional alcohol.

Preferably said extraction solution consists of approximately from 29.9 to 69.9% by weight water, approximately from 29.9 to 69.9% by weight of at least one monofunctional alcohol selected from a group comprising ethyl alcohol, propyl alcohol, approximately from 29.9 to 69.9% by weight glycerine and approximately from 0.001% to 0.1% by weight of said biochemical salt.

In a preferred version said extraction solution consists of approximately 29.9% by weight of water, approximately 29.9% by weight of a monofunctional alcohol selected from a group comprising ethyl alcohol, propyl alcohol, approximately 29.9% by weight of glycerine and approximately from 0.001% to 0.1% by weight of said biochemical salt.

Preferably said monofunctional alcohol is selected from a group comprising ethyl alcohol, propyl alcohol.

Preferably said at least one alcohol comprises only a polyfunctional alcohol, preferably a trifunctional alcohol, in a greatly preferred manner glycerine.

In this way, an extraction solution containing only water, biochemical salt and trifunctional alcohol is generated.

This version is particularly suitable for use in nutraceutic compositions for use by consumers of paediatric age or intolerant to certain alcoholic compounds.

In a preferred version said solvent comprises approximately from 30 to 70% by weight water and approximately from 30 to 70% by weight polyfunctional alcohol. A nutraceutic composition suitable for use by users of paediatric age is obtained.

In a preferred version said extraction solution consists of approximately from 29.9 to 69.9% by weight water and approximately from 29.9 to 69.9% by weight polyfunctional alcohol. In this way, a nutraceutic composition suitable for use by users of paediatric age is obtained.

In a preferred version said solvent comprises from 30 to 35% by weight water and from 30 to 35% by weight monofunctional alcohol and from 30 to 35% by weight polyfunctional alcohol.

In a preferred version said solvent comprises from 30 to 35% by weight water and from 30 to 35% by weight monofunctional alcohol and from 30 to 35% by weight glycerine.

In a particularly preferred version said solvent comprises approximately from 33% by weight water and 33% by weight monofunctional alcohol and 33% by weight glycerine.

The Applicant has verified that such solvent composition allows to further enhance the extraction of the desired bioactive compounds from the plant meristems.

By modulating the relative amounts of water, ethyl alcohol and glycerine it is possible to modulate the quantity and/or the type of bioactive compounds extracted from the plant meristems and that can be found in the nutraceutic composition.

In one version said method comprises selecting a desired biochemical salt based on the plant of said plant meristem and/or based on the characteristics desired for said nutraceutic composition.

The Applicant verified that the effectiveness of the extraction of the active compounds from the various plant meristems varied depending on the particular biochemical salt dissolved in the extraction solution. Therefore, the Applicant verified that an extraction solution containing a single biochemical salt could allow to enhance the effectiveness of the extraction of the active compounds from the plant meristems. Furthermore, the Applicant verified that by appropriately selecting the biochemical salt based on the plant meristem it was possible to enhance the extraction of the active compounds in particular of certain and desired bioactive compounds contained in the meristem and, therefore, to obtain a bud-derivative having particular desired properties. By selecting the biochemical salt to be added to the plant meristem, it is possible to modulate the properties of the derived bud obtained.

In a preferred version said solvent consists of one third water, one third ethyl alcohol and one third glycerine.

The Applicant has verified that with these values of the proportions between water, glycerine and ethyl alcohol it is possible to improve the extraction of the bioactive compounds, improving the selectivity of the extraction, favouring the extraction of the desired bioactive compounds and reducing the extraction of undesired compounds.

Preferably it is provided for immersing a quantity of said at least one plant meristem in said solution so as to obtain an admixture having a weight ratio meristem: extraction solution between 1:3 and 1:20, preferably between 1:10 and 1:20, in a greatly preferred manner between 1:15 and 1:20.

In a preferred version it is provided for immersing a quantity of said at least one plant meristem in said extraction solution so as to obtain an admixture having a weight ratio meristem: extraction solution of approximately 1:20.

The Applicant has verified that increasing the extraction ratio between solvent and plant meristem improves the extraction of the bioactive composites from the plant meristems. In particular, the Applicant has verified that a weight ratio meristem: extraction solution between approximately from 1:15 to 1:20 is optimal qualitatively.

These values of the weight ratio between meristem: extraction solution are calculated considering the dry content of the plant meristems used.

With the values of weight ratio meristem: extraction solution of the invention there is a higher extraction efficiency: with a higher solvent ratio, there is more solvent available for dissolving the active ingredients from the plant matrix. A more complete extraction of the bioactive compounds is obtained and a more concentrated nutraceutic composition is obtained.

The Applicant has verified that with the values of weight ratio meristem: extraction solution of the invention a better extraction selectivity is obtained, favouring the extraction of the desired bioactive compounds and reducing the extraction of undesired compounds.

With the values of weight ratio meristem: extraction solution of the invention, a lower risk of degradation of the plant meristems during maceration is obtained.

Furthermore, according to the invention it is not provided for cutting or damaging the plant meristems before extraction. This, together with the presence of the desired Schüssler salt in the extraction solution, allows to enhance the properties of the nutraceutic composition obtained.

Preferably the plant meristems are immersed intact in the extraction solution.

Therefore, longer maceration times can be used.

Preferably, said method comprises a step of preparing said extraction solution comprising providing a solvent containing from 30 to 70% by weight water and from 30 to 70% by weight of at least one monofunctional alcohol selected from ethyl alcohol and propyl alcohol, dissolving in said solvent approximately from 0.001% to 0.1% by weight of said biochemical salt. Preferably said method further comprises a step of solubilizing said biochemical salt in said solvent for a solubilization period between 20 and 30 hours, preferably approximately 24 hours.

Preferably said method comprises homogenizing said salt and said solvent by means of an immersion homogenizer at a speed between 10,000 and 33,000 rpm during said solubilizing so as to obtain an intermediate solution.

Preferably said method comprises adding to said intermediate solution at least one polyfunctional alcohol so as to obtain an extraction solution containing approximately from 29.9 to 49.9% by weight water, approximately from 29.9 to 49.9% by weight of at least one monofunctional alcohol, approximately from 29.9 to 49.9% by weight of at least one polyfunctional alcohol, preferably glycerine, and approximately from 0.001% to 0.1% by weight of said salt a biochemical salt.

In a preferred version said extraction solution comprises approximately from 29.9 to 34.9% by weight water, approximately from 29.9 to 34.9% by weight of at least one monofunctional alcohol, approximately from 29.9 to 34.9% by weight of at least one polyfunctional alcohol, preferably glycerine, and approximately from 0.001% to 0.1% by weight of said biochemical salt.

. In a particularly preferred version said extraction solution comprises approximately 33% by weight water and 33% by weight monofunctional alcohol and 33% by weight glycerine and approximately from 0.001% to 0.1% by weight of said biochemical salt. Preferably said method comprises mixing said polyfunctional alcohol to said intermediate solution so as to obtain said extraction solution containing glycerine, water, monofunctional alcohol and biochemical salt.

Preferably said method comprises a balancing step of balancing said intended extraction solution after immersing said at least one plant meristem in said extraction solution. Said balancing step comprises measuring the quantity of water in said admixture.

Preferably said method comprises a measuring step in which the quantity of water contained in the plant meristems is measured.

Preferably said method comprises a calculation step in which the quantity of water to be used to obtain an extraction solution containing approximately from 29.9 to 34.9% by weight water, approximately from 29.9 to 34.9% by weight of at least one monofunctional alcohol, approximately from 29.9 to 34.9% by weight of at least one polyfunctional alcohol, preferably glycerine, and approximately from 0.001% to 0.1% by weight of said biochemical salt is calculated.

In a preferred version said balancing step comprises adding a desired quantity of water and/or of said at least one alcohol to said admixture so as to vary the water/alcohol ratios and obtain an extraction solution containing approximately from 29.9 to 69.9% by weight water, approximately from 29.9 to 69.9% by weight of said at least one alcohol.

In a preferred embodiment said balancing step comprises adding a desired quantity of water, and/or of said at least one monofunctional alcohol and/or of said at least one polyfunctional alcohol to said admixture so as to balance the composition of said extraction solution and obtain an extraction solution containing approximately from 29.9 to 49.9% by weight water, approximately from 29.9 to 49.9% by weight of said at least one monofunctional alcohol, approximately from 29.9 to 49.9% by weight of said at least one polyfunctional alcohol.

Preferably said balancing step comprises adding a desired quantity of water, and/or of said at least one monofunctional alcohol and/or of said at least one polyfunctional alcohol to said admixture so as to balance the composition of said extraction solution and obtain an extraction solution containing approximately from 29.9 to 34.9% by weight water, approximately from 29.9 to 34.9% by weight of at least one monofunctional alcohol, approximately from 29.9 to 34.9% by weight of at least one polyfunctional alcohol, preferably glycerine, and approximately from 0.001% to 0.1% by weight of said biochemical salt.

In this way it is possible to compensate for any variations in the water content in the plant meristems, balancing the water content present in the plant meristem and at the same time avoiding the formation of mixtures that are too diluted or, conversely, too concentrated.

Preferably said method comprises collecting said plant meristem.

Preferably said immersion step is provided within approximately 2 hours of the collection step of said meristem.

Preferably said immersion step provides for immersing said intact plant meristem into said extraction solution. Preferably, it is not provided to treat the plant meristem before immersing it in the extraction solution, in particular to cut or chop the plant meristem.

Preferably said maceration step is carried out for a time between approximately 2 weeks and approximately 4 weeks, preferably approximately 3 weeks.

Preferably said maceration step is carried out for approximately 21 days.

Preferably said method comprises a final decanting step in which it is provided for decanting said admixture at a pressure between 120 and 200 kg/cm² for a decanting time period between approximately 24 and approximately 48 hours.

Preferably, said decanting step is carried out by means of a controlled pressure press. This decanting step allows to avoid damage to the embryonic tissues of the plant meristems.

In some versions, the decantation step is provided before the filtration step.

In other versions, the decantation step is provided after the filtration step.

Preferably said decantation step is provided after said maceration step.

Preferably said method comprises, at the end of the maceration step and of said decantation step, a step of filtering said admixture to separate the solid fraction of said admixture from the liquid fraction of said admixture. The solid fraction is constituted by the solid parts of the plant meristems, the liquid fraction by the water, the solvent, the bioactive composites extracted from the plant meristem and by the biochemical salt dissolved in the liquid fraction. The liquid fraction corresponds to the nutraceutic composition.

Preferably said method provides for the use of water at a pH between 5.8 and 6.2, preferably approximately 6. Preferably low mineral water is used. Preferably water having a hardness of approximately 0.44 French degrees is used, preferably with bicarbonate ionic base and calcium.

Preferably said monofunctional alcohol is ethyl alcohol obtained from the fermentation of beets.

Preferably said trifunctional alcohol is glycerine obtained from red palm. Said glycerine preferably contains tocotrienol.

Preferably said plant meristem is selected from a group comprising buds, young buds, lymphs, catkins.

Preferably said plant meristem belongs to a plant selected from a group comprising
- *Abies pectinata DC*
- *Betula pubescens Ehrh.*
- *Hippophae rhamnoides L.*
- *Rosa canina L.*
- *Ribes nigrum L.*
- *Prunus spinosa L. and Quercus robur L.*
- *Tamarix gallica L.*
- *Tilia tomentosa Moench*
- *Salix alba L.*
- *Juglans regia L.*
- *Betula pendula Roth and Fraxinus excelsior L.*
- *Betula pendula Roth*

Preferably said plant meristem consists of buds of *Abies pectinata DC.*

Preferably said plant meristem consists of buds and catkins of *Betula pubescens Ehrh.*

Preferably said plant meristem consists of buds of *Hippophae rhamnoides L.*

Preferably, said plant meristem consists of young buds of *Rosa canina L.*

Preferably said plant meristem consists of buds of *Ribes Nigrum L.*

Preferably said plant meristem consists of buds of *Prunus spinosa L.* and buds of *Quercus robur L* in analogous parts.

Preferably said plant meristem consists of young buds of *Tamarix gallica L.*

Preferably said plant meristem consists of buds of *Tilia tomentosa Moench*

Preferably said plant meristem consists of buds of *Salix alba L.*

Preferably said plant meristem consists of buds of *Juglans regia L.*

Preferably said plant meristem consists of lymph of *Betula pendula Roth* and of buds of *Fraxinus excelsior L.* in analogous parts.

Preferably said plant meristem consists of buds of *Betula pendula Roth.*

Preferably said plant meristem is a plant meristem coming from only one type of plant.

Preferably a nutraceutic composition is provided containing bioactive compounds from plant meristems of buds of Abies pectinata DC with Calcium fluoratum CaF₂. This nutraceutic composition is particularly suitable as a regulator of calcium metabolism, and favours the elasticity of the tissues and at the same time their structure.

Preferably a nutraceutic composition is provided containing bioactive compounds from plant meristems of catkins and/or buds of Betula pubescens Ehrh. with Calcium phoshoricum, CaHPO₄. This nutraceutic composition is particularly suitable as a reconstituent, musculoskeletal reinforcement and synthesis of red and white blood cells and as an endocrine support.

Preferably a nutraceutic composition is provided containing bioactive compounds from plant meristems of buds of Hippophae rhamnoides L. with Calcium sulphuricum, CaSO₄. Such a composition is particularly suitable for immune regulation, hyperactivity and loss of adaptive capacity.

Preferably a nutraceutic composition is provided containing bioactive compounds from plant meristems of young buds of Rosa canina L. with Ferrum phoshoricum, Fe₄(P₂O₇)₃. This composition is particularly suitable for the treatment of acute local or systemic inflammation, fever, inflammation, diarrhoea, muscle trauma, strains.

Preferably a nutraceutic composition is provided containing bioactive compounds from plant meristems of buds of Ribes nigrum L. with Kalium muriaticum (Kalium chloratum, KCI). This composition is particularly suitable for the treatment of inflammation.

Preferably a nutraceutic composition is provided containing bioactive compounds from plant meristems of buds of Prunus spinosa L. and buds of Quercus robur L. with Kalium phoshoricum, K₃PO₄. This composition is particularly suitable for the treatment of poor nervous and hormonal reactivity, immunosuppression, fatigue, asthenia, atonia, anxious-depressive states.

Preferably a nutraceutic composition is provided containing bioactive compounds from plant meristems of young buds of Tamarix gallica L. with Kalium sulphuricum, K₃SO₄. This composition has microbial and antioxidant function and is particularly suitable in the treatment of convalescence of retrovirus infections by improving immunological function.

Preferably a nutraceutic composition is provided containing bioactive compounds from plant meristems of buds of Tilia tomentosa Moench with Magnesium phoshoricum, MgHPO₄. This composition is particularly suitable as anxiolytic, antispastic, sympatholytic.

Preferably a nutraceutic composition is provided containing bioactive compounds from plant meristems of buds of Salix alba L. with Natrium muriaticum, NaCl. This composition has a vagotonic, anabolic function and favours tissue trophism and regulates the extracellular compartment.

Preferably a nutraceutic composition is provided containing actives from plant meristems of buds of Juglans regia L. with Natrium phoshoricum, Na₃PO₄. This composition is particularly suitable in the treatment of pancreatic trophism, dyspepsia, acidosis, and as a dewormer.

Preferably a nutraceutic composition is provided containing bioactive compounds from plant meristems of lymph of Betula pendula Roth and actives from plant meristems of buds Fraxinus excelsior L. with Natrium sulphuricum, Na₂SO₄. This composition is particularly suitable as a diuretic, anti-oedemic, uricosuric and favours diuresis, renal drainage and adrenal regulation.

Preferably a nutraceutic composition is provided containing bioactive compounds from plant meristems of buds of Betula pendula Roth with Silica (Silicea), SiO₂. This composition has a remineralizing, immunostimulating function and is particularly suitable in the treatment of mucosal hypertrophy, chronic inflammation, chronic infections.

Preferably said plant meristem comes from Abies pectinata DC and said biochemical salt is Calcium fluoratum, calcium fluoride CaF₂.

Preferably said plant meristem comes from Betula pubescens Ehrh. and said biochemical salt is Calcium phoshoricum, calcium acid phosphate CaHPO₄

Preferably said plant meristem comes from Hippophae rhamnoides L. and said biochemical salt is Calcium sulphuricum, Calcium sulphate CaSO₄.

Preferably said plant meristem comes from Rosa canina L. and said biochemical salt is Ferrum phoshoricum, iron pyrophosphate Fe₄(P₂O₇)₃.

Preferably said plant meristem comes from Ribes nigrum L. and said biochemical salt is Kalium muriaticum, potassium chloride KCl;.

Preferably said plant meristem comes from Prunus spinosa L. and from Quercus robur L. and said biochemical salt is Kalium phoshoricum, potassium phosphate K₃PO₄.

Preferably said plant meristem comes from Tamarix gallica L. and said biochemical salt is Kalium sulphuricum, potassium sulphate K₂SO₄;.

Preferably said plant meristem comes from Tilia tomentosa Moench buds and said biochemical salt is Magnesium phoshoricum, magnesium phosphate MgHPO4.

Preferably said plant meristem comes from Salix alba L. and said biochemical salt is Natrium muriaticum, sodium chloride NaCl.

Preferably said plant meristem comes from Juglans regia L. and said biochemical salt is Natrium phoshoricum, sodium phosphate Na₃PO₄.

Preferably said plant meristem comes from Betula pendula Roth and from Fraxinus excelsior L. and said biochemical salt is Natrium sulphuricum, sodium sulphate Na₂SO₄.

Preferably said plant meristem comes from Betula pendula Roth and said biochemical salt is Silica (Silicea), silicon dioxide SiO₂.

It should be noted that some steps of the methods described above may be independent of the order of execution reported. In addition, some steps may be optional. In addition, some steps of the methods may be performed repetitively, or they may be performed in series or in parallel with other steps of the method.

The characteristics and advantages of the invention will become clearer from the detailed description of a preferred embodiment thereof, shown by way of non-limiting example, with reference to the appended drawings, wherein:

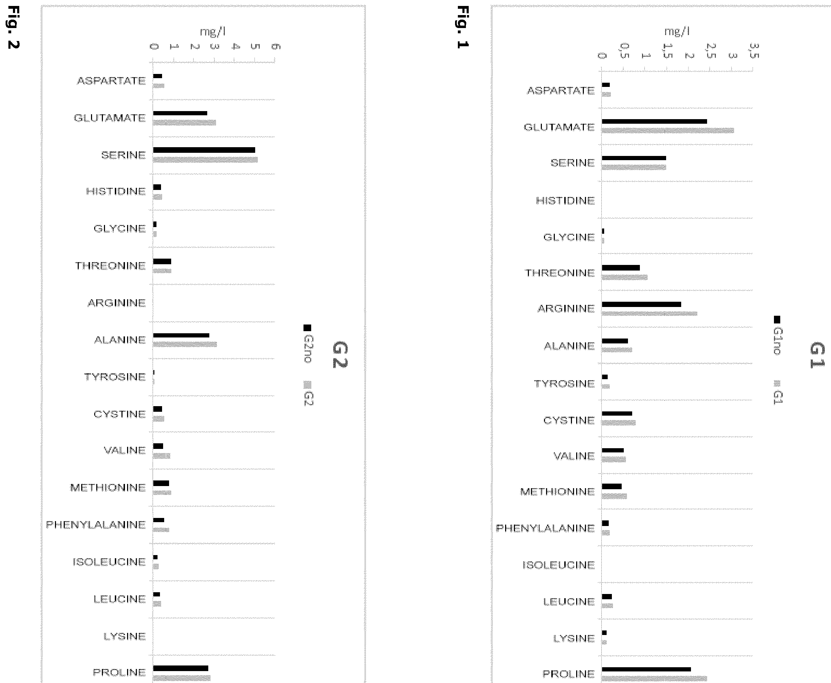

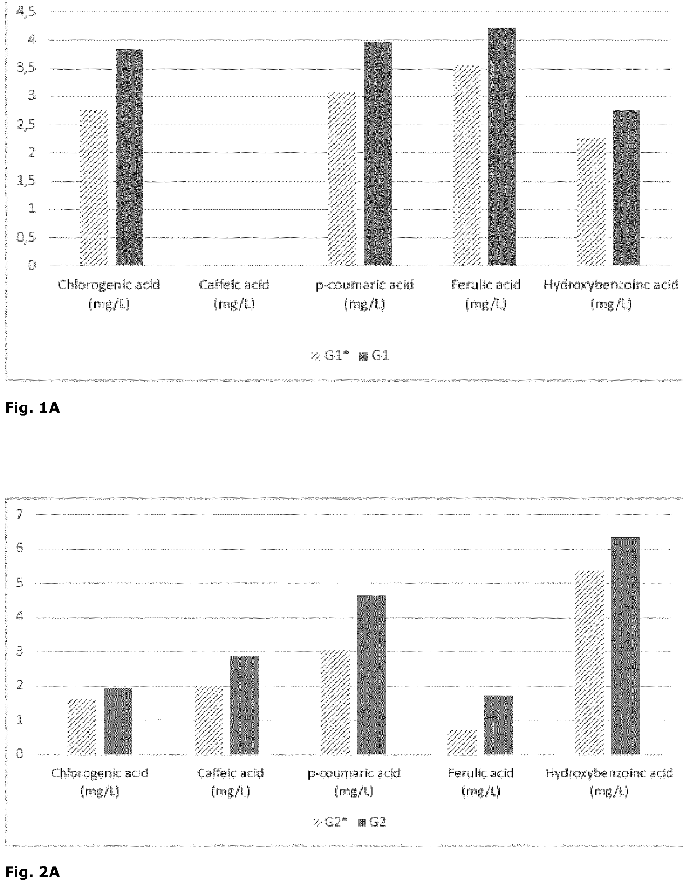

Figure 1 is a graph showing the concentration values of some amino acids in the nutraceutic composition G1 obtained from plant meristems (buds) of Abies pectinata DC with calcium fluoratum according to the invention and the concentration values of a nutraceutic composition obtained from plant meristems of Abies pectinata DC according to the known technique G1no.
Figure 1A is a graph showing the concentration values of some phenolic acids in the nutraceutic composition G1 obtained from plant meristems (buds) of Abies pectinata DC(White Fir) with calcium fluoratum according to the invention (G1) and the values of the same acids obtained without addition of salt to the extraction solution (G1no). Figure 2 is a graph showing the concentration values of some amino acids in the nutraceutic composition G2 obtained from plant meristems (catkins and/or buds) of Betula pubescens Ehrh. E. with calcium phosphoricum according to the invention and the concentration values of a nutraceutic composition obtained from plant meristems of Betula pubescens Ehrh. according to the known technique G2no. Figure 2A is a graph showing the concentration values of some phenolic acids in the nutraceutic composition G2 obtained from plant meristems (catkins and/or buds) of Betula pubescens Ehrh. E. with calcium phosphoricum according to the invention (G2) and the values of the same phenolic acids obtained without addition of salt to the extraction solution (G2no).

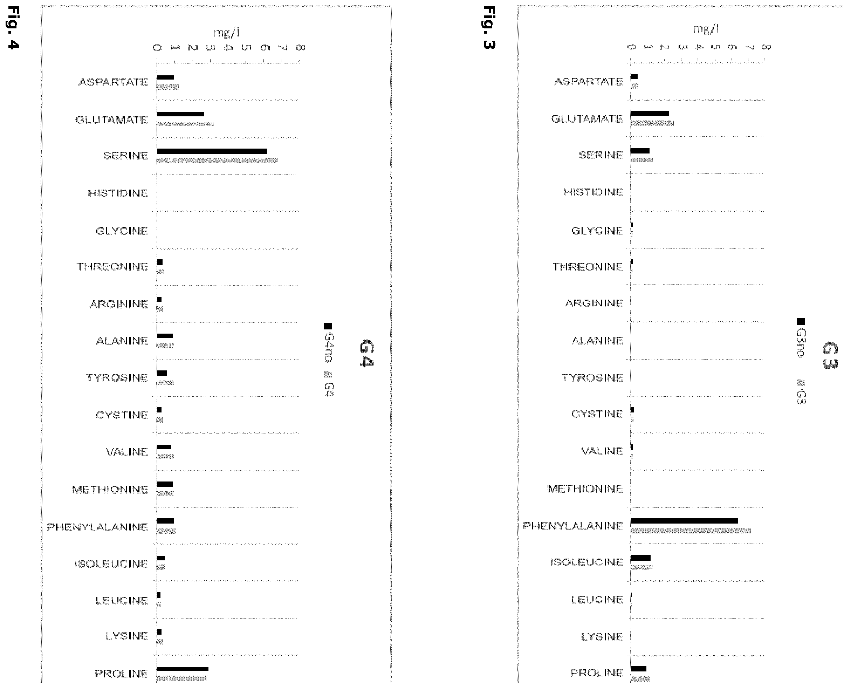

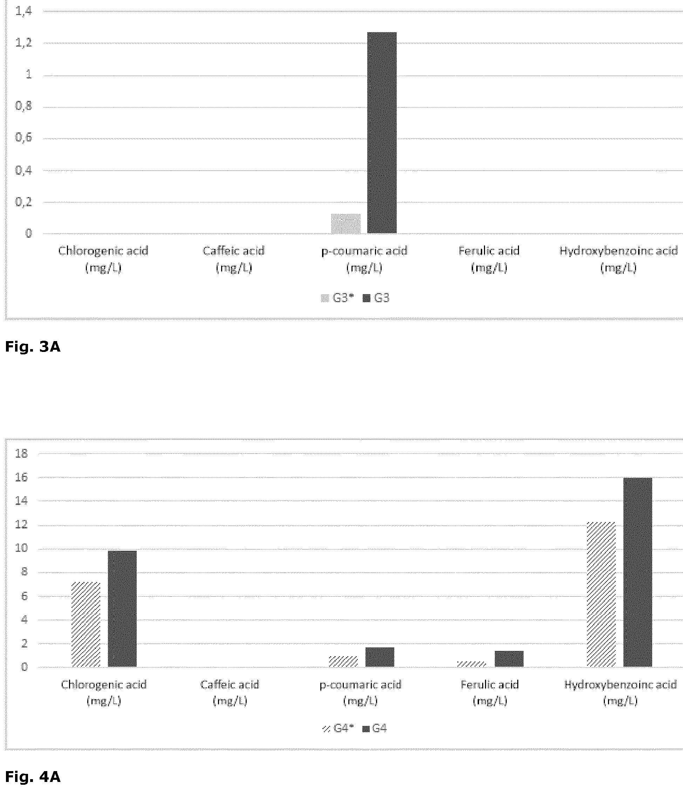

Figure 3 is a graph showing the concentration values of some amino acids in the nutraceutic composition G3 obtained from plant meristems (buds) of Hippophae rhamnoides (Sea buckthorn) with Calcium sulphuricum according to the invention and the concentration values of a nutraceutic composition obtained from plant meristems of Hippophae rhamnoides according to the known technique G3no.
Figure 3A is a graph showing the concentration values of some phenolic acids in the nutraceutic composition G3 obtained from plant meristems (buds) of Hippophae rhamnoides (Sea buckthorn) with Calcium sulphuricum according to the invention (G3) and the values of the same phenolic acids obtained without addition of salt to the extraction solution (G3no).
Figure 4 is a graph showing the concentration values of some amino acids in the nutraceutic composition G4 obtained from plant meristems (young buds) of Rosa canina L. with Ferrum phoshoricum according to the invention and the concentration values of a nutraceutic composition obtained from plant meristems of Rosa canina L. according to the known technique G4no.
Figure 4A is a graph showing the concentration values of some phenolic acids in the nutraceutic composition G4 obtained from plant meristems (young buds) of Rosa canina L with Ferrum phoshoricum according to the invention (G4) and the values of the same phenolic acids obtained without addition of salt to the extraction solution (G4no).

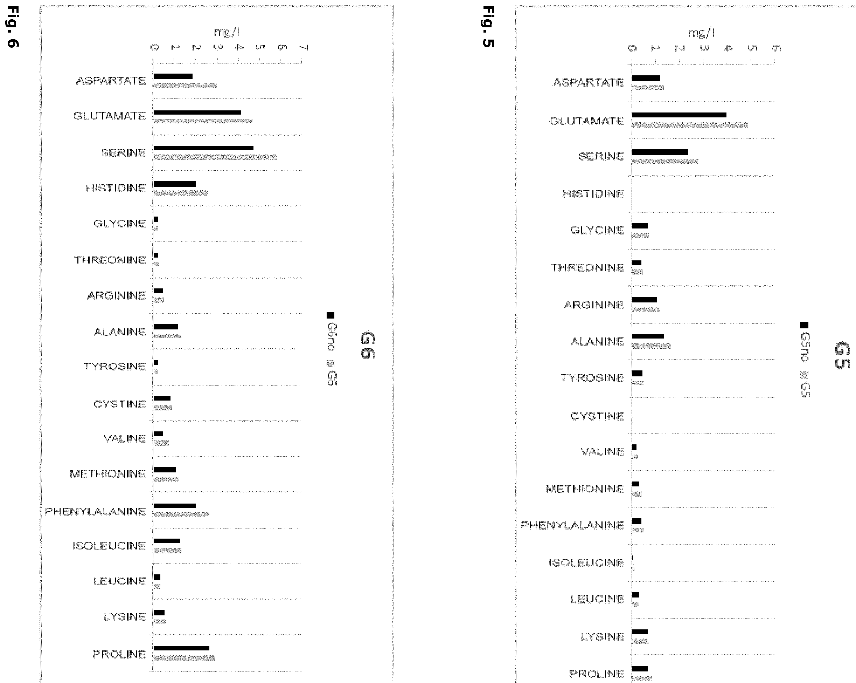

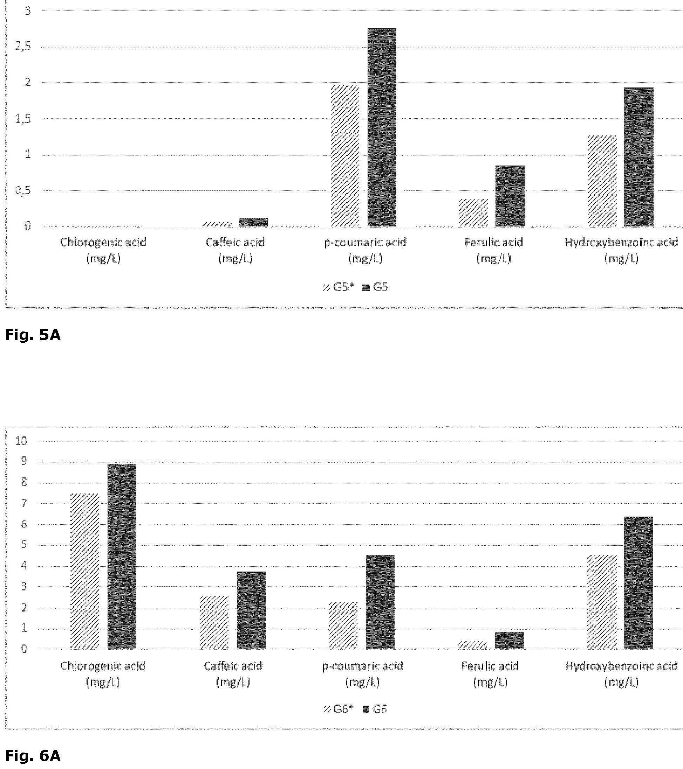

Figure 5 is a graph showing the concentration values of some amino acids in the nutraceutic composition G5 obtained from plant meristems (buds) of Ribes nigrum L. (Black Ribes) with Kalium muriaticum and the concentration values of a nutraceutic composition obtained from plant meristems of buds of Ribes nigrum L. according to the known technique G5no.
Figure 5A is a graph showing the concentration values of some phenolic acids in the nutraceutic composition G5 obtained from plant meristems (buds) of Ribes nigrum L. with Kalium muriaticum (G5) and the values of the same amino acids obtained without addition of salt to the extraction solution (G5no).
Figure 6 is a graph showing the concentration values of some amino acids in the nutraceutic composition G6 obtained from meristems (buds) of Prunus spinosa L. (Wild Prunus) and (buds) of Quercus robur L. with Kalium phoshoricum and the concentration values of a nutraceutic composition obtained from plant meristems (buds) of Prunus spinosa L. and (buds) of Quercus robur L. G6no;
Figure 6A is a graph showing the concentration values of some amino acids in the nutraceutic composition G6 obtained from meristems (buds) of Prunus spinosa L. (Wild Prunus) and (buds) of Quercus robur L. with Kalium phoshoricum according to the invention and the values of the same amino acids obtained without addition of salt to the extraction solution (G6no);

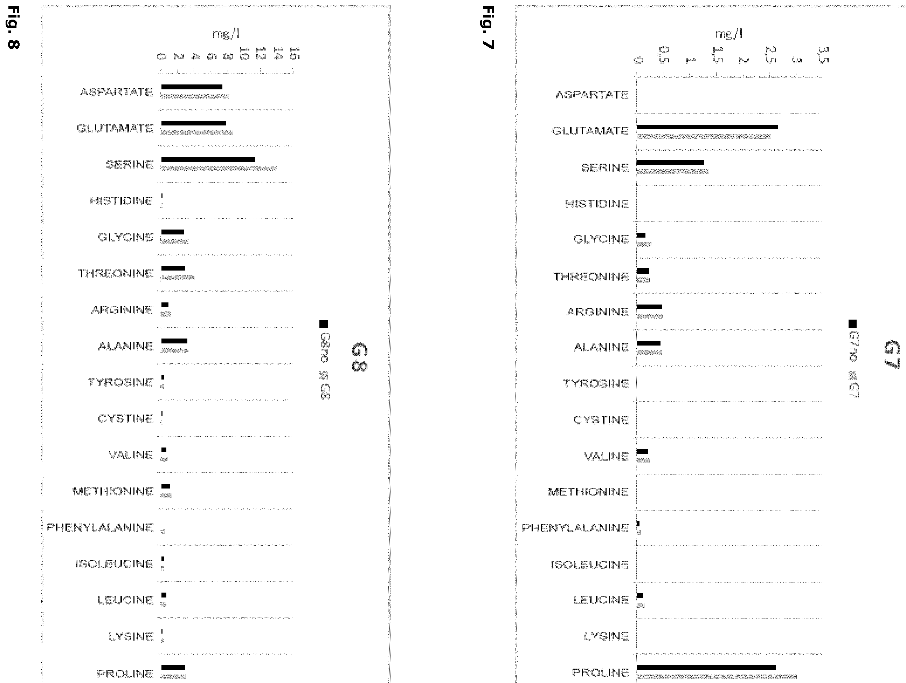

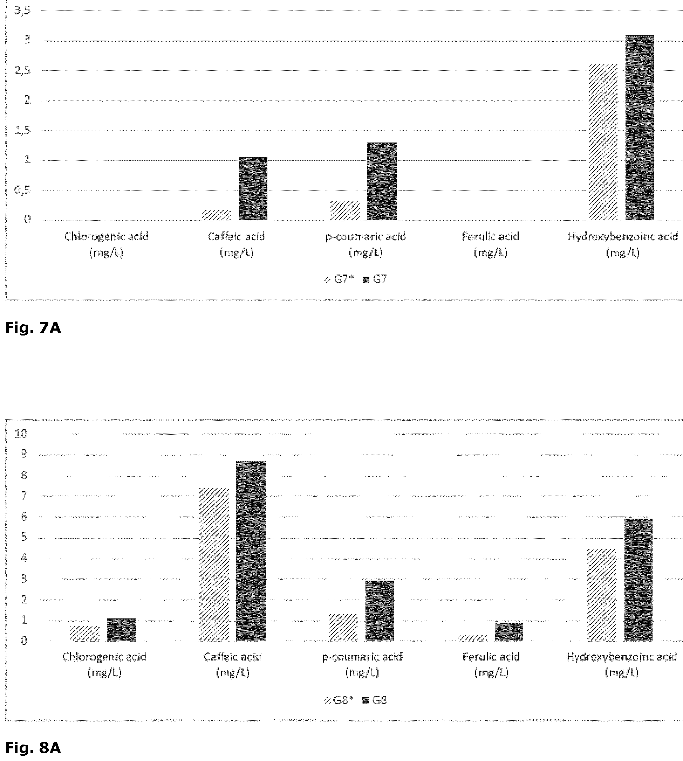

Figure 7 is a graph showing the concentration values of some amino acids in the nutraceutic composition G7 obtained from plant meristems (young buds) of Tamarix gallica L. (Common tamarix) with Kalium sulphuricum and the concentration values of a nutraceutic composition obtained from plant meristems (young buds) of Tamarix gallica L. G7no;
Figure 7A is a graph showing the concentration values of some amino acids in the nutraceutic composition G7 obtained from plant meristems (young buds) of Tamarix gallica L. (Common tamarix) with Kalium sulphuricum according to the invention (G7) and the values of the same amino acids obtained without addition of salt to the extraction solution (G7no);
Figure 8 is a graph showing the concentration values of some amino acids in the nutraceutic composition G8 obtained from plant meristems (buds) of Tilia tomentosa Moench (Silver Lime) with Magnesium phoshoricum. and the concentration values of amino acids in the nutraceutic composition obtained from plant meristems (buds) of Tilia tomentosa Moench G8no.
Figure 8A is a graph showing the concentration values of some phenolic acids in the nutraceutic composition G8 obtained from plant meristems (buds) of Tilia tomentosa Moench (Silver Lime) with Magnesium phoshoricum according to the invention (G8) and the values of the same phenolic acids obtained without addition of salt to the extraction solution (G8no);

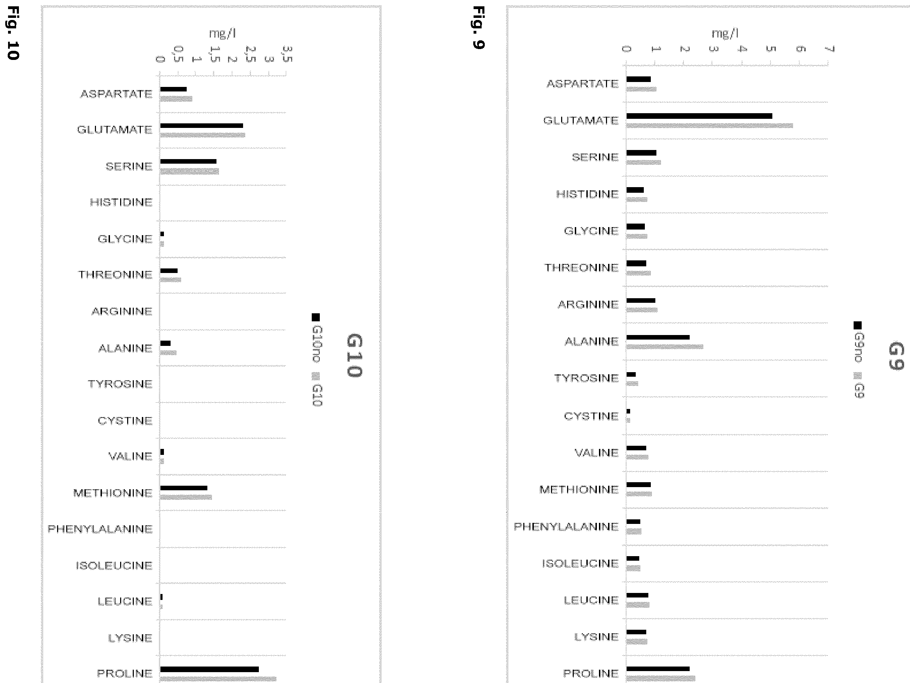

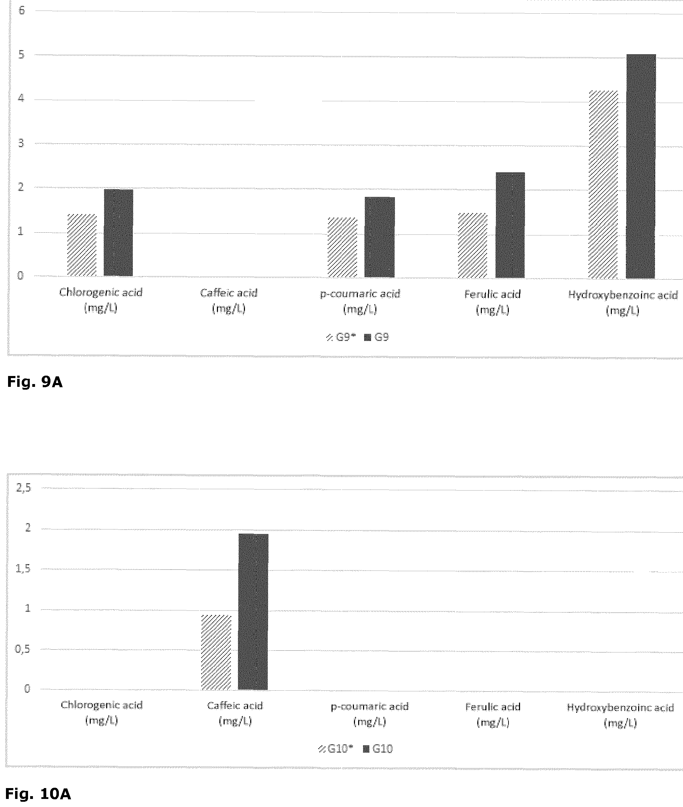

Figure 9 is a graph showing the concentration values of some amino acids in the nutraceutic composition G9 obtained from plant (buds) meristems (buds) of Salix alba L. with Natrium muriaticum and the concentration values of amino acids in the nutraceutic composition obtained from plant meristems (buds) of Salix alba L. G9no.
Figure 9A is a graph showing the concentration values of some phenolic acids in the nutraceutic composition G9 obtained from plant (buds) meristems (buds) of Salix alba L. with Natrium muriaticum according to the invention (G9) and the values of the same phenolic acids obtained without addition of salt to the extraction solution (G9no);
Figure 10 is a graph showing the concentration values of some amino acids in the nutraceutic composition G10 obtained from plant (buds) meristems (buds) of Juglans regia L. with Natrium phoshoricum and the concentration values of amino acids in the nutraceutic composition obtained from plant meristems (buds) of Juglans regia L. G10no.
Figure 10A is a graph showing the concentration values of some phenolic acids in the nutraceutic composition G10 obtained from plant (buds) meristems (buds) of Juglans regia L. with Natrium phoshoricum according to the invention (G10) and the values of the same phenolic acids obtained without addition of salt to the extraction solution (G10no);

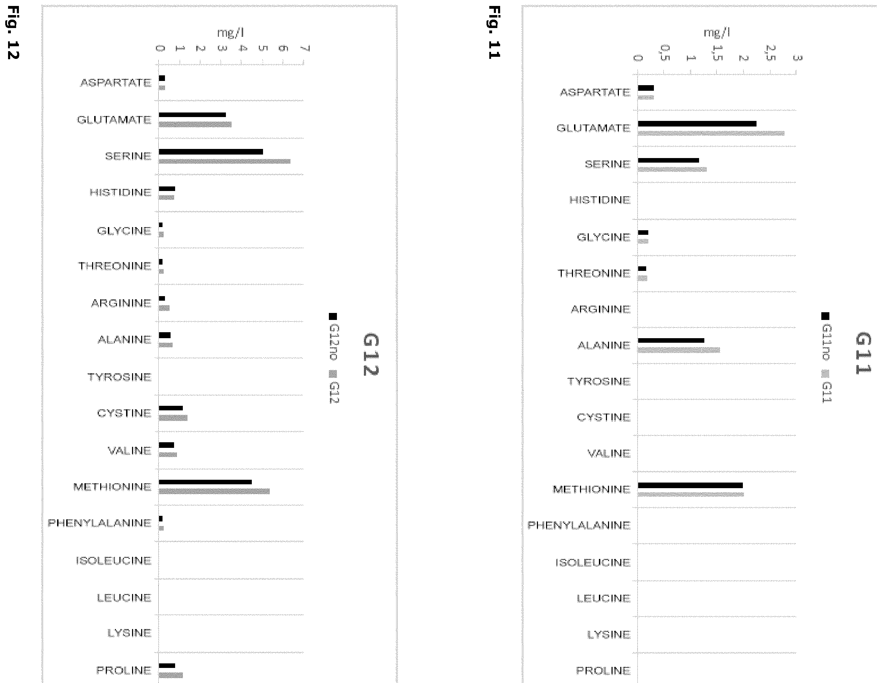

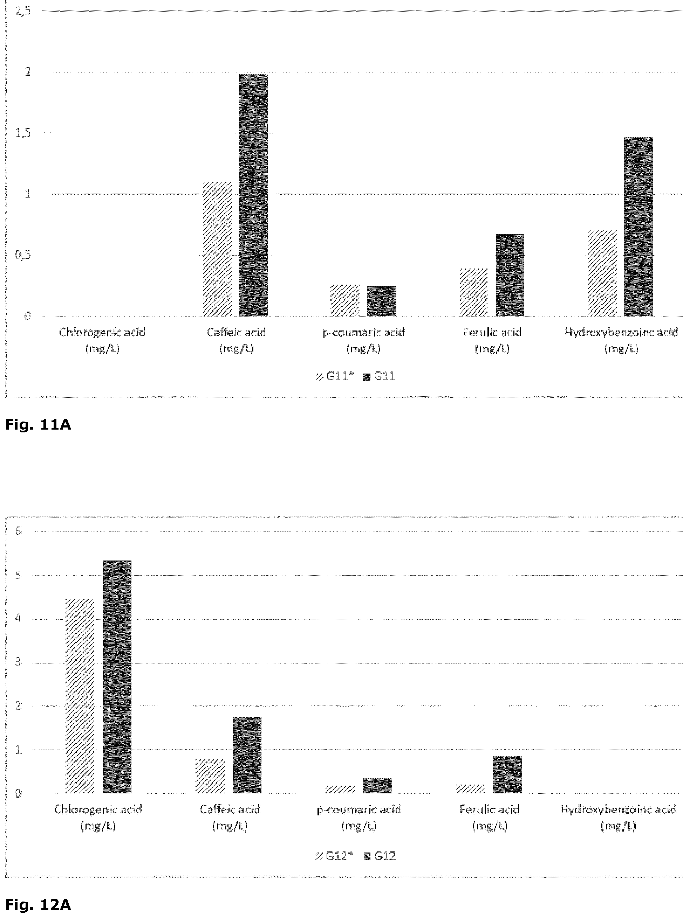

Figure 11 is a graph showing the concentration values of some amino acids in the nutraceutic composition G11 obtained from plant meristems (lymph) of Betula pendula Roth (buds) (White birch) of Fraxinus excelsior L. (Ash) with Natrium sulphuricum and the concentration values of amino acids in the nutraceutic composition obtained from plant meristems (lymph) of Betula pendula Roth (buds) of Fraxinus excelsior L. G11no.
Figure 11a is a graph showing the concentration values of some phenolic acids in the nutraceutic composition G11 obtained from plant meristems (lymph) of Betula pendula Roth (buds) (White birch) of Fraxinus excelsior L. (Ash) with Natrium sulphuricum according to the invention (G11) and the values of the same phenolic acids obtained without addition of salt to the extraction solution (G11no);
Figure 12 is a graph showing the concentration values of some amino acids in the nutraceutic composition G12 obtained from plant meristems (buds) of Betula pendula Roth. with silica and the concentration values of amino acids in the nutraceutic composition obtained from plant meristems (buds) of Betula pendula Roth G12no.
Figure 12a is a graph showing the concentration values of some phenolic acids in the nutraceutic composition G12 obtained from plant meristems (buds) of Betula pendula Roth. with silica according to the invention (G12) and the values of the same phenolic acids obtained without addition of salt to the extraction solution (G12no);

It is to be noted that the amino acid concentration values shown in Figures 1-12 were obtained with high performance liquid chromatography (HPLC) analysis using a C18 reverse-phase column. The analyses were carried out in triplicate to validate the results obtained.

### KEY TO FIGURES

**G1:** nutraceutic composition obtained from plant meristems (buds) of Abies pectinata DC with Calcium fluoratum.

**G1no:** nutraceutic composition obtained from plant meristems (buds) of Abies pectinata DC according to the known technique, without adding Calcium Fluoratum.

**G2:** nutraceutic composition obtained from plant meristems (catkins and/or buds) of Betula pubescens Ehrh. with Calcium phoshoricum.

**G2no:** nutraceutic composition obtained from plant meristems (catkins and/or buds) of Betula pubescens Ehrh according to the known technique, without addition of Calcium Fluoratum.

**G3:** nutraceutic composition obtained from plant meristems (buds) of Hippophae rhamnoides. with Calcium sulphuricum.

**G3no:** nutraceutic composition obtained from plant meristems (buds) of Hippophae rhamnoides, without addition of Calcium sulphuricum.

**G4:** nutraceutic composition obtained from plant meristems (young buds) of Rosa canina L. with Ferrum phoshoricum.

**G4no:** nutraceutic composition obtained from plant meristems (young buds) of Rosa canina L, without addition of Ferrum phoshoricum.

**G5:** nutraceutic composition obtained from plant meristems (buds) of Ribes nigrum L. with Kalium muriaticum.

**G5no:** nutraceutic composition obtained from plant meristems (buds) of Ribes nigrum L. without addition of Kalium muriaticum.

**G6:** nutraceutic composition obtained from plant meristems (buds) of Prunus spinosa L. and (buds) of Quercus robur L. with Kalium phoshoricum.

**G6no:** nutraceutic composition obtained from plant meristems (buds) of Prunus spinosa L. and (buds) of Quercus robur L. without addition of Kalium phoshoricum.

**G7:** nutraceutic composition obtained from plant meristems (young buds) of Tamarix gallica L. with Kalium sulphuricum.

**G7no:** nutraceutic composition obtained from plant meristems (young buds) of Tamarix gallica L. without addition of Kalium sulphuricum.

**G8:** nutraceutic composition obtained from plant meristems (buds) of Tilia tomentosa Moench with Magnesium phoshoricum.

**G8no:** nutraceutic composition obtained from plant meristems (buds) of Tilia tomentosa Moench without addition of Magnesium phoshoricum.

**G9:** nutraceutic composition obtained from plant meristems (buds) of Salix alba L. with Natrium muriaticum.

**G9no:** nutraceutic composition obtained from plant meristems (buds) of Salix alba L. without addition of Natrium muriaticum. **G10:** nutraceutic composition obtained from plant meristems (buds) of Juglans regia L. with Natrium phoshoricum.

**G10no:** nutraceutic composition obtained from plant meristems (buds) of Juglans regia L. without addition of Natrium phoshoricum.

**G11:** nutraceutic composition obtained from plant meristems (lymph) of Betula pendula Roth (buds) of Fraxinus excelsior L. with Natrium sulphuricum.

**G11no:** nutraceutic composition obtained from plant meristems (lymph) of Betula pendula Roth (buds) of Fraxinus excelsior L. without addition of Natrium sulphuricum.

**G12:** nutraceutic composition obtained from plant meristems (buds) of Betula pendula Roth with Silica (Silicea).

**G12no:** nutraceutic composition obtained from plant meristems (buds) of Betula pendula Roth without addition of Silica (Silicea).

### EXAMPLES

### Example 1

A nutraceutic composition based on buds and catkins of Betula pubescens and calcium acid phosphate CaHPO4, (named **G2**) was prepared with a method described below.

The buds and catkins of Betula pubescens were collected.

The percentage of water present on the collected bud-derivatives was calculated by means of a drying scale. The water present in the meristem is considered in order to evaluate the quantity of water to be introduced as a solvent. In this case, 300 grams of water were calculated to be present in one kg of bud-derivative.

An extraction solution was prepared by mixing in a mixer with a stirring speed of approximately 20,000 rpm 6.37 kg of water at a pH of approximately 6 and a hardness of 0.44 French degrees and 6.67 kg of ethyl alcohol obtained from beet fermentation. The aforementioned quantity of water will eventually allow to have a total of (6.37+ 0.3)= 6.67 Kg of water.

While keeping stirring the mixer, 0.05 g of calcium acid phosphate CaHPO4 were added to the water and ethyl alcohol to obtain the intermediate solution. The intermediate solution is kept under constant stirring for 24 hours. At the end of 24 hours, 6.67 kg of plant glycerine from red palm is added to the intermediate solution of CaHPO4 in water and ethyl alcohol. In this way approximately 20 kg of the desired extraction solution containing the desired solvent (water, alcohol, glycerine) and the specific biochemical salt are obtained.

20 kg of extraction solution are inserted into a steel silo where a quantity of fresh meristems corresponding to 1 kg of dried plant is then added. The plant meristems are added to the extraction solution within 2 hours of their collection.

The quantity of plant meristems to be added to the extraction solution is calculated so as to obtain an extraction solution: dry plant ratio between approximately 15:1 and 20:1. The water content present in the plant meristems is calculated in order to balance the content of the extraction solution and obtain an extraction solution containing approximately 33% water, approximately 33% ethyl alcohol and approximately 33% glycerine and the selected biochemical salt.

The 20:1 ratio calculated on the weight of the dry part (dry content) of the plant meristems.

The plant meristems are left to macerate in the extraction solution for approximately 21 days.

The admixture obtained by means of controlled pressure is allowed to decant through a customised press so as not to break the embryonic tissues with a pressure of 200 Kg/cm².

It is provided for letting the decanted admixture rest for approximately 40 hours.

Subsequently, it is provided for filtering the admixture through a filter to remove the decanted solid part and any floating residues.

The filtered liquid part constitutes the nutraceutic composition of the invention having a high content of bioactive compounds.

### Comparative example 1

A nutraceutic composition based on plant meristems (buds) of Betula pubescens was also prepared according to the method described in Example 1 with the exception of the step of addition of a biochemical salt. The step of addition of a biochemical salt was therefore avoided. For the rest, the same parameters and procedures as in Example 1 were adopted, avoiding, as mentioned, only the dissolution of calcium acid phosphate CaHPO4.

The composition of Example 1 is indicated in the figures with G2, the nutraceutic composition of the Comparative Example 1 is indicated in the figures with G2no. From the comparison of the amino acid content of the two nutraceutic compositions it is noted that the method of the invention allows to obtain nutraceutic compositions having improved properties compared to the known nutraceutic compositions.

It is possible to note that thanks to the addition of an appropriate biochemical salt, selected from among the biochemical Salts based on the bud-derivative to be used, it is possible to obtain higher concentrations of the amino acids contained in the bud-derivative.

Indeed, the chromatographic analyses shown in Figure 2 reveal that the amino acid composition of Betula pubescens buds and catkins exhibits higher concentrations in the nutraceutic composition obtained according to the method of the invention (G2) than the lower concentrations of the amino acid composition of Betula pubescens buds and catkins obtained without the use of calcium acid phosphate (G2no).

The analyses reported in Figure 2A show that the addition of the calcium acid phosphate CaHPO4 allows to increase the extraction of the phenolic acids contained in the buds and catkins of Betula pubescens, and to obtain a nutraceutic composition having higher concentrations of phenolic acids. The nutraceutic composition of the invention therefore has, under other conditions, higher concentrations of phenolic acids than that obtained without the use of calcium acid phosphate.

### Example 2

A nutraceutic composition based on buds of Prunus spinosa and Quercus robur with the addition of potassium phosphate K3PO4 (named G6) was moreover prepared with a method described below.

The buds of Prunus spinosa and Quercus robur were collected.

The percentage of water present on the collected bud-derivatives was calculated by means of a drying scale. The water present in the meristems is considered in order to calculate the quantity of water to be introduced as a solvent. In this case, 450 grams of water were calculated to be present in one kg of bud-derivative.

An extraction solution was prepared by mixing in a mixer with a stirring speed of approximately 30,000 rpm, 13.6 kg of water at a pH of approximately 6 and a hardness of 0.44 French degrees and 6.5 kg of plant glycerine from red palm. This quantity of water will eventually allow to have a total of (13.6+0.45) = approximately 14 kg of water.

While keeping stirring, 0.1 g of potassium phosphate K3PO4 was added.

The extraction solution obtained is transferred to a steel silo in which approximately 500 grams of bud-derivatives of Prunus spinosa and approximately 500 grams of Quercus robur are added. The bud-derivatives should preferably be introduced within 2 hours since their collection.

The plant meristems were left to macerate in the extraction solution for 21 days.

The admixture obtained was decanted by means of controlled pressure through a customised press so as not to break the embryonic tissues with a pressure of 200 Kg/cm².

The decanted admixture was allowed to rest for approximately 40 hours.

Subsequently, the admixture was filtered through a filter to remove the decanted solid part and any floating residues.

The filtered liquid part constitutes the nutraceutic composition of the invention having a high content of bioactive compounds.

### Comparative example 2

A nutraceutic composition based on plant meristems (of Prunus spinosa and Quercus robur buds) was moreover prepared according to the method described in Example 2 with the exception of the step of addition of a biochemical salt. The step of addition of a biochemical salt was therefore avoided. For the rest, the same parameters and procedures as in Example 2 were adopted, avoiding, as mentioned, only the dissolution of potassium phosphate K₃PO₄.

The composition of Example 2 is indicated in the figures with G6, the nutraceutic composition of Comparative Example 2 is indicated in the figures with G6no. From the comparison of the amino acid content of the two nutraceutic compositions it is noted that the method of the invention allows to obtain nutraceutic compositions having improved properties compared to the known nutraceutic compositions. -

It is possible to note that thanks to the addition of an appropriate biochemical salt, selected from among the biochemical Salts based on the bud-derivative to be used, it is possible to obtain higher concentrations of the amino acids contained in the bud-derivative.

In fact, the chromatographic analyses shown in Figure 6 reveal that the amino acid composition of the nutraceutic composition G6 based on Prunus spinosa and Quercus robur with potassium phosphate has higher concentrations in the nutraceutic composition obtained according to the method of the invention than the lower concentrations of the amino acid composition of the nutraceutic composition G6 based on Prunus spinosa and Quercus robur obtained without the use of potassium phosphate.

Similarly Figure 6A shows that the nutraceutic composition of the invention has a higher concentration of phenolic acids than the corresponding nutraceutic composition obtained without addition of potassium phosphate.

### FURTHER EXAMPLES

With the same methodology as described above, nutraceutic compositions containing plant meristems according to the invention were prepared as better described below. The corresponding nutraceutic compositions were then obtained by using an extraction solution free of biochemical salt.

An extraction solution containing water, ethyl alcohol and glycerine and possibly a biochemical salt was used.

In all cases, the meristem was introduced into the extraction solution within two hours since collection.

The plant meristem was left to macerate in the extraction solution for a time of approximately 3 weeks. At the end of this period, the admixture obtained was decanted by means of controlled pressure through a customised press so as not to break the embryonic tissues with a pressure of 200 Kg/cm². The decanted admixture was allowed to rest for approximately 40 hours. Subsequently, the admixture was filtered through a filter to remove the decanted solid part and any floating residues. The filtered liquid part constitutes the nutraceutic composition of the invention having a high content of bioactive compounds.

The nutraceutic compositions obtained were analysed to identify the content and the concentration of amino acids and phenolic acids, obtaining the results reported in the figures.

The following nutraceutic compositions have been prepared:
G1 obtained from plant meristems, buds, of Abies pectinata DC with Calcium fluoratum (G1), G1no obtained from plant meristems, buds, of Abies pectinata DC and without adding Calcium Fluoratum to the extraction solution.

**G2:** nutraceutic composition obtained from plant meristems (catkins and/or buds) of Betula pubescens Ehrh. with Calcium phoshoricum, **G2no:** nutraceutic composition obtained from plant meristems (catkins and/or buds) of Betula pubescens Ehrh according to the known technique, without addition of Calcium Fluoratum.

**G3:** nutraceutic composition obtained from plant meristems (buds) of Hippophae rhamnoides. with Calcium sulphuricum, **G3no:** nutraceutic composition obtained from plant meristems (buds) of Hippophae rhamnoides, without addition of Calcium sulphuricum.

**G4:** nutraceutic composition obtained from plant meristems (young buds) of Rosa canina L. with Ferrum phoshoricum, **G4no:** nutraceutic composition obtained from plant meristems (young buds) of Rosa canina L, without addition of Ferrum phoshoricum.

**G5:** nutraceutic composition obtained from plant meristems (buds) of Ribes nigrum L. with Kalium muriaticum, **G5no:** nutraceutic composition obtained from plant meristems (buds) of Ribes nigrum L. without addition of Kalium muriaticum.

**G6:** nutraceutic composition obtained from plant meristems (buds) of Prunus spinosa L. and (buds) of Quercus robur L. with Kalium phoshoricum, **G6no:** nutraceutic composition obtained from plant meristems (buds) of Prunus spinosa L. and (buds) of Quercus robur L. without addition of Kalium phoshoricum.

**G7:** nutraceutic composition obtained from plant meristems (young buds) of Tamarix gallica L. with Kalium sulphuricum, **G7no:** nutraceutic composition obtained from plant meristems (young buds) of Tamarix gallica L. without addition of Kalium sulphuricum.

**G8:** nutraceutic composition obtained from plant meristems (buds) of Tilia tomentosa Moench with Magnesium phoshoricum, **G8no:** nutraceutic composition obtained from plant meristems (buds) of Tilia tomentosa Moench without addition of Magnesium phoshoricum. **G9:** nutraceutic composition obtained from plant meristems (buds) of Salix alba L. with Natrium muriaticum, **G9no:** nutraceutic composition obtained from plant meristems (buds) of Salix alba L. without addition of Natrium muriaticum.

**G10:** nutraceutic composition obtained from plant meristems (buds) of Juglans regia L. with Natrium phoshoricum, **G10no:** nutraceutic composition obtained from plant meristems (buds) of Juglans regia L. without addition of Natrium phoshoricum.

**G11:** nutraceutic composition obtained from plant meristems (lymph) of Betula pendula Roth (buds) of Fraxinus excelsior L. with Natrium sulphuricum, **G11no:** nutraceutic composition obtained from plant meristems (lymph) of Betula pendula Roth (buds) of Fraxinus excelsior L. without addition of Natrium sulphuricum.

**G12:** nutraceutic composition obtained from plant meristems (buds) of Betula pendula Roth with Silica (Silicea), **G12no:** nutraceutic composition obtained from plant meristems (buds) of Betula pendula Roth without addition of Silica (Silicea).

Figures 1-12 show that the nutraceutic compositions prepared according to the method of the invention have an amino acid content with concentrations higher than the concentrations that can be found in the corresponding compositions known in the art.

Figures 1A-12A show that the nutraceutic compositions prepared according to the method of the invention have a higher phenolic acid content than the concentrations that can be found in the corresponding compositions obtained without addition of the corresponding biochemical salt.

The data of Figures 1A-12A are collected in Table 1 below.

| **No. SAMPLE** | **MERISTEM** | **BIOCHEMICAL SALT** | **Chlorogenic acid (mg/L)** | **Caffeic acid (mg/L)** | **p-coumaric acid (mg/L)** | **Ferulic Acid (mg/L)** | **Hydroxyb enzoinc Acid (mg/L)** | **total phenolic acids (mg/L)** |
|---|---|---|---|---|---|---|---|---|
| **G1no** | SPRUCE BUDS | | 2.75068 | 0 | 3.07722 | 3.55702 | 2.26574 | **2.330132** |
| **G1** | SPRUCE BUDS | CALCIUM FLORIDE | 3.84580 | 0 | 3.98494 | 4.23285 | 2.76420 | **2.965560** |
| **G2no** | BETULA PUBESCENS CATKINS AND BUDS | | 1.614456 | 2.01064 | 3.0776 | 0.714172 | 5.38722 | **12.80409** |
| **G2** | BETULA PUBESCENS CATKINS AND BUDS | CALCIUM PHOSPHATE | 1.927467 | 2.89426 | 4.63157 | 1.721305 | 6.37207 | **17.54667** |
| **G3no** | SEA BUCKTHORN BUDS | | 0 | 0 | 0.130527 | 0 | 0 | **0.13053** |
| **G3** | SEA BUCKTHORN BUDS | CALCIUM SULPHATE | 0 | 0 | 1.26924 | 0 | 0 | **1.26924** |
| **G4no** | ROSA CANINA YOUNG BUDS | | 7.25976 | 0 | 1.020186 | 0.546386 | 12.23828 | **21.06461** |
| **G4** | ROSA CANINA YOUNG BUDS | IRON PHOSPHATE | 9.83487 | 0 | 1.730267 | 1.435672 | 15.95467 | **28.95548** |
| **G5no** | BLACKCURRANT BUDS | | 0 | 0.0635648 | 1.96474 | 0.391184 | 1.272898 | **3.69239** |
| **G5** | BLACKCURRANT BUDS | POTASSIUM CHLORIDE | 0 | 0.1232562 | 2.753420 | 0.845326 | 1.934258 | **5.65626** |
| **G6no** | WILD BLACKTHORN, OAK BUDS | | 7.49358 | 2.5857 | 2.27994 | 0.377146 | 4.5746 | **17.31097** |
| **G6** | WILD BLACKTHORN, OAK BUDS | POTASSIUM PHOSPHATE | 8.89543 | 3.74353 | 4.553420 | 0.845326 | 6.39543 | **24.43314** |
| **G7no** | TAMARIX YOUNG BUDS | | 0 | 0.1794162 | 0.310156 | 0 | 2.61238 | **3.10195** |
| **G7** | TAMARIX YOUNG BUDS | POTASSIUM SULPHATE | 0 | 1.04852 | 1.292152 | 0 | 3.08954 | **5.43021** |
| **G8no** | LINDEN BUDS | | 0.73492 | 7.41816 | 1.333996 | 0.301958 | 4.4492 | **14.23823** |
| **G8** | LINDEN BUDS | MAGNESIUM PHOSPHATE | 1.12328 | 8.73182 | 2.933235 | 0.930182 | 5.94074 | **19.65926** |
| **G9no** | WHITE WILLOW BUDS | | 1.405592 | 0 | 1.354702 | 1.48778 | 4.27782 | **8.52589** |
| **G9** | WHITE WILLOW BUDS | SODIUM CHLORIDE | 1.97232 | 0 | 1.838255 | 2.390975 | 5.08507 | **11.28662** |
| **G10no** | WALNUT BUDS | | 0 | 0.942796 | 0 | 0 | 0 | **0.94280** |
| **G10** | WALNUT BUDS | SODIUM PHOSPHATE | 0 | 1.954637 | 0 | 0 | 0 | **1.95464** |
| **G11no** | WHITE BIRCH LYMPH ASH BUDS | | 0 | 1.100498 | 0.257246 | 0.39438 | 0.70876 | **2.46088** |
| **G11** | WHITE BIRCH LYMPH ASH BUDS | SODIUM SULPHATE | 0 | 1.985363 | 0.247245 | 0.673945 | 1.470847 | **4.37740** |
| **G12no** | WHITE BIRCH BUDS | | 4.46332 | 0.804668 | 0.196797 | 0.221768 | 0 | **5.68655** |
| **G12** | WHITE BIRCH BUDS | SILICA | 5.346332 | 1.784536 | 0.377245 | 0.867394 | 0 | **8.37551** |

The addition of a biochemical salt in the concentrations of the invention to the extraction solution allows to enhance the extraction of the bioactive compounds from the plant meristems. At the same time, the meristem/solvent proportion in the range of the invention allows the extraction of the bioactive composites to be enhanced.

Moreover, the Applicant has verified that the addition of a desired specific biochemical salt to a particular plant meristem in order to obtain the above-mentioned plant meristem/biochemical salt combinations allows to favour the extraction of the bioactive compounds contained in the plant meristem. The Applicant has verified that specific combinations of one or more specific biochemical salts with plant meristems coming from one or more specific plants allows for high synergy and that this allows to maximise the extraction of one or more amino acid composites. This thus enhances the properties of the nutraceutic compositions obtained.

## Claims

1. Method for obtaining a nutraceutic composition comprising the step of providing an extraction solution containing approximately from 29.9 to 69.9% by weight of water and approximately from 29.9 to 69.9% by weight of at least one alcohol which is selected from a group comprising ethyl alcohol, propyl alcohol, glycerine or the combinations thereof and approximately from 0.001% to 0.1% by weight of a biochemical salt, said biochemical salt being selected from a group comprising:
*Salt no. 1 Calcium fluoratum -* calcium fluoride, CaF₂;
Salt no. 2 *Calcium phosphoricum -* calcium acid phosphate CaHPO₄;
Salt no. 3 *Calcium sulfuricum -* Calcium sulphate CaSO₄
Salt no. 4 *Ferrum phosphoricum -* iron pyrophosphate Fe₄(P₂O₇)₃;
Salt no. 5 *Kalium chloratum -* potassium chloride KCl;
Salt no. 6 *Kalium phosphoricum -* potassium phosphate K₃PO₄;
Salt no. 7 *Kalium sulfuricum -* potassium sulphate K₂SO₄;
Salt no. 8 *Magnesium phosphoricum* - magnesium phosphate MgHPO₄;
Salt no. 9 *Natrium chloratum* - sodium chloride NaCl;
Salt no. 10 *Natrium phosphoricum -* sodium phosphate Na₃PO₄;
Salt no. 11 *Natrium sulfuricum -* sodium sulphate Na₂SO₄;
Salt no. 12 Silicea - silicon dioxide SiO₂;
- immersing in said extraction solution a quantity of at least one plant meristem so as to obtain an admixture having a weight ratio of meristem: extraction solution between approximately 1:3 and approximately 1:20, preferably between approximately 1:15 and 1:20;
- leaving said plant meristems to macerate in said extraction solution for a maceration period between 20 hours and 4 weeks, preferably between approximately 2 weeks and approximately 3 weeks so as to extract one or more bioactive compounds from said at least one plant meristem;
- filtering said admixture so as to obtain a nutraceutic composition comprising between approximately from 30 to 69.6% by weight of water, approximately from 30 to 69.6% by weight of said at least one alcohol, approximately from 0.001 to 0.1% by weight of said salt and approximately from 0.3 to 5% by weight of said one or more bioactive compounds.

2. Method according to the preceding claim wherein said alcohol comprises at least one monofunctional alcohol and at least one polyfunctional alcohol, said monofunctional alcohol being selected from a group comprising ethyl alcohol, propyl alcohol, said polyfunctional alcohol being selected from a group comprising glycerine, preferably said alcohol comprises a monofunctional alcohol, such as for example ethyl alcohol or propyl alcohol, and glycerine.

3. Method according to the preceding claim wherein said solvent comprises approximately from 30 to 35% by weight water and approximately from 30 to 35% by weight monofunctional alcohol and approximately from 30 to 35% by weight polyfunctional alcohol.

4. Method according to claim 2 or 3, wherein said method comprises preparing an extraction solvent comprising from 29.9 to 69.9% by weight water and approximately from 29.9 to 69.9% by weight of at least one alcohol selected from a group comprising ethyl alcohol, propyl alcohol, glycerine and dissolving in said solvent approximately from 0.001% to 0.1% by weight of at least one biochemical salt in order to obtain said extraction solution.

5. Method according to one of the preceding claims wherein said extraction solvent consists of approximately from 30 to 35% by weight water, approximately from 30 to 35% by weight monofunctional alcohol selected from ethyl alcohol or propyl alcohol and approximately from 30 to 35% by weight glycerine.

6. Method according to the preceding claim wherein said extraction solution consists of said extraction solvent and approximately from 0.001% to 0.1% by weight of a biochemical salt selected from Schüssler Salts.

7. Method according to one of the preceding claims wherein said step of preparing said extraction solution comprises providing a solvent containing from 30 to 70% by weight water and from 30 to 70% by weight of at least one monofunctional alcohol selected from ethyl alcohol and propyl alcohol, dissolving in said solvent approximately from 0.001% to 0.1% by weight of said biochemical salt, solubilizing said biochemical salt in said solvent for a solubilization period between 20 and 30 hours, preferably approximately 24 hours, said method preferably comprising homogenizing said biochemical salt and said solvent at a rate between 10,000 and 33,000 rpm in order to obtain an intermediate solution, said method preferably further comprising adding to said intermediate solution at least one polyfunctional alcohol so as to obtain an extraction solution containing approximately from 29.9 to 49.9% by weight water, approximately from 29.9 to 49.9% by weight of at least one monofunctional alcohol, approximately from 29.9 to 49.9% by weight of at least one polyfunctional alcohol, preferably glycerine, and approximately from 0.001% to 0.1% by weight of said biochemical salt.

8. Method according to one of the preceding claims wherein said immersing said at least one plant meristem in said solution comprises immersing a quantity of said at least one plant meristem so as to obtain an admixture having a weight ratio meristem:extraction solution between approximately 1:5 and approximately 1:20, preferably between approximately 1:10 and approximately 1:20, in a greatly preferred manner between approximately 1:15 and approximately 1:20, said weight ratio being calculated considering the dry portion of said meristem.

9. Method according to one of the preceding claims and further comprising decanting said admixture at a pressure between approximately 120 and approximately 200 kg/cm² for a decanting time period between approximately 24 and approximately 48 hours.

10. Method according to one of the preceding claims wherein when said plant meristem is plant lymph, said admixture has a weight ratio meristem:extraction solution between approximately 1:3 and approximately 1:5, preferably approximately 1:3.33, and wherein when said plant meristem is a solid plant meristem, said admixture has a weight ratio meristem: extraction solution between approximately 1:3 and approximately 1:20.

11. Method according to one of the preceding claims wherein said plant meristem is selected from buds, young buds, lymphs, catkins; said plant meristem belonging to a plant selected from a group comprising:
- *Abies pectinata DC*
- *Betula pubescent*
- *Hippophae rham L.*
- *Rosa canina L.*
- *Ribes nigrum L.*
- *Prunus spin L. and Quercus rob.L*
- *Tamarix gallica L. giovani*
- *Tilia tomentosa M.*
- *Salix alba L.*
- *Juglans regia L.*
- *Betula pendula Roth (linfa) Fraxinus ex. L.*
- *Betula pendula Roth*

12. Nutraceutic composition obtained with the method according to one of the preceding claims comprising between approximately from 29.9 to 69.6% by weight of water, approximately from 29.9 to 69.6% by weight of said at least one alcohol, approximately from 0.001 to 0.1% by weight of a biochemical salt and approximately from 0.3 to 5% by weight of one or more bioactive compounds, said biochemical salt being selected from a group comprising:
- *Salt no. 1 Calcium fluoratum -* calcium fluoride, CaF2;
- Salt no. 2 *Calcium phosphoricum -* calcium acid phosphate CaHPO4;
- Salt no. 3 *Calcium sulfuricum -* Calcium sulphate CaSO4
- Salt no. 4 *Ferrum phosphoricum -* iron pyrophosphate Fe₄(P₂O₇)₃;
- Salt no. 5 *Kalium chloratum -* potassium chloride KCl;
- Salt no. 6 *Kalium phosphoricum -* potassium phosphate K3PO4;
- Salt no. 7 *Kalium sulfuricum -* potassium sulphate K2SO4;
- Salt no. 8 *Magnesium phosphoricum* - magnesium phosphate MgHPO4;
- Salt no. 9 *Natrium chloratum* - sodium chloride NaCl;
- Salt no. 10 *Natrium phosphoricum -* sodium phosphate Na3PO4;
- Salt no. 11 *Natrium sulfuricum -* sodium sulphate Na2SO4;
- Salt no. 12 Silicea - silicon dioxide SiO2.
